# EUROPEAN PATENT APPLICATION

(11) **EP 1 609 836 A1**
(43) Date of publication of application: **28.12.2005**
(21) Application number: 04724428.0
(22) Date of filing: 30.03.2004
(51) Int. Cl.: C09K 3/00, A61K 7/00, A61L 9/01

(54) **GEL-STATE COMPOSITION**

(30) Priority: 01.04.2003 JP 2003098195
(71) Applicant: Ajinomoto Co., Inc., Tokyo 104-8315 (JP)
(72) Inventor: YAMATO, Naoya c/o Ajinomoto Co., Inc., Kawasaki-shi, Kanagawa 210-8681 (JP)
(74) Representative: Hart Davis, Jason
(86) International application number: PCT/JP2004/004568
(87) International publication number: WO 2004/090063

(57) **Abstract**

A gel-state composition useful as, e.g., a composition for perfume cosmetics which is translucent to milk-white, is creamy to solid, has an excellent appearance, gives neither a tacky feeling nor a cooped-up feeling during use, and has excellent spreadability. The gel-state composition is **characterized by** containing at least one N-acylamino acid (A) represented by the general formula (1) and at least one oily base (B):

R₁-CO-X (1)

(In the formula, R₁ represents a C₁₋₂₅, linear or branched, saturated hydrocarbon group or an unsaturated hydrocarbon group optionally containing an aromatic ring; and X represents an aliphatic or aromatic α-, β-, or γ-amino acid.)

## Description

### Technical Field

The present invention relates to a gel composition comprising a prescribed N-acyl amino acid and oily base, and in addition thereto, an optional higher aliphatic acid. In the gel composition of the present invention, the oily base is gelled to achieve a variety of forms, permitting ready use in aromatic cosmetic compositions such as cosmetics, aromatics, and the like, and achieving good organoleptic aspects.

### Technical Background

Condensates of polyhydric alcohols and aromatic aldehydes typified by polyamide resins, 12-hydroxystearic acid, and dibenzylidene-D-sorbitol are known as gelling agents for water-insoluble oily bases. However, there are problems in that the gel compositions for aromatic cosmetics in which these gelling agents are employed exhibit poor solubility, the gel compositions for aromatic cosmetics become nonuniform, and a "sweating phenomenon" occurs in which the gelled oily base undergoes change over time and exudes out onto the surface of the gel.

N-acyl-L-glutamic acid dibutyl amide has been proposed as a gelling agent for oily bases (see Patent References 1 and 2). It is known to gel various oily bases. However, the gelled oily base obtained is a relatively solid gel, and when the gel composition is applied to the skin as a cosmetic, the formulation is frail, there are problems with robustness, and the applications are limited. There are further problems in the form of low solubility in common oily bases and the requirement of relatively high temperatures to solubilize this gelling agent.

Antiperspirant gel sticks containing 12-hydroxystearic acid and N-lauroyl-L-glutamic acid dibutyl amide have been proposed (see Patent References 3 and 4). However, the gelled oily bases are relatively solid gels, the formulations are frail, there are problems with robustness, and applications are limited.

The use of fatty acid dextrin esters as gelling agents to gel oily bases has also been proposed (see Patent Reference 5). However, all formulations employ high gelling agent levels of 10 weight percent or more, and since the gelling agent itself is a macromolecular compound of relatively high molecular weight, there is an undesirably strong filmy sensation when employed on the skin or hair. Silicone oils cannot be gelled by fatty acid dextrin esters, so the oily bases that can be gelled are limited.
[Patent Reference 1] Japanese Patent Application Publication No. Showa 51-19139
[Patent Reference 2] Japanese Patent Application Publication No. 2002-316971
[Patent Reference 3] U.S. Patent No. 5,591,424
[Patent Reference 4] International Patent Publication in Japanese No. Heisei 7-506833
[Patent Reference 5] Japanese Patent Application Publication No. Heisei 08-047635

### Description of the Invention

### [Problem to Be Solved by the Invention]

The object of the present invention is to solve the above-described problems of prior art by providing a gel composition that is useful as a composition for aromatic cosmetics (that is, compositions blending well as starting materials into cosmetics, aromatics, and other aromatic cosmetics), that is translucent to milky white, is creamy to solid, is attractive in appearance, does not produce a clammy or clogging sensation when used, and spreads well.

### [Means of Solving the Problem]

The present inventors conducted extensive research into solving the above-stated problems. As a result, they discovered that a gel composition consisting of at least one component selected from among prescribed N-acyl amino acids and one component selected from among oily bases, as well as such gel compositions further containing a higher aliphatic acid, were translucent to milky white, creamy to solid, attractive in appearance, and pleasing to the touch; the present invention was devised on this basis.

That is, the present invention may assume the following configurations:
(1) A gel composition characterized by comprising at least one N-acyl amino acid (A) denoted by general formula (1) below and at least one oily base (B): 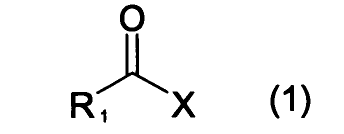 (wherein R₁ denotes a straight or branched-chain saturated hydrocarbon group having from 1 to 25 carbon atoms or an unsaturated hydrocarbon group optionally containing an aromatic ring and X denotes an aliphatic or aromatic α-, β-, or γ-amino acid).
(2) The gel composition of (1) above further, characterized in that an optically active form (L-form or D-form) of N-acyl amino acid (A) is employed.
(3) The gel composition of (1) or (2) above, further characterized in that the X in general formula (1) of N-acyl amino acid (A) denotes a neutral amino acid.
(4) The gel composition of any of (1) to (3) above, further characterized in that N-acyl amino acid (A) is N-acyl-L-alanine.
(5) The gel composition of any of (1) to (4) above, further characterized in that in N-acyl amino acid (A) of general formula (1), R₁ denotes a straight or branched-chain saturated hydrocarbon group with 5 to 21 carbon atoms, or an unsaturated hydrocarbon group optionally comprising an aromatic ring.
(6) The gel composition of any of (1) to (5) above, further characterized in that in N-acyl amino acid (A) of general formula (1), R₁ denotes a straight or branched-chain aliphatic hydrocarbon group with 7 to 17 carbon atoms.
(7) The gel composition of any of (1) to (6) above, further characterized in that N-acyl amino acid (A) is selected from among N-2-ethylhexanoyl-L-alanine, N-capryloyl-L-alanine, N-caproyl-L-alanine, N-lauroyl-L-alanine, N-myristoyl-L-alanine, and N-palmitoyl-L-alanine.
(8) The gel composition of any of (1) to (7) above, further characterized in that N-acyl amino acid (A) is an N-lauroyl amino acid.
(9) The gel composition of any of (1) to (8) above, further characterized in that N-acyl amino acid (A) is N-lauroyl-L-alanine.
(10) The gel composition of any of (1) to (9) above, further characterized in that oily base (B) is liquid at room temperature.
(11) The gel composition of any of (1) to (10) above, further characterized in that oily base (B) is a hydrocarbon oil or silicone oil.
(12) The gel composition of any of (1) to (11) above, further characterized in that oily base (B) is a silicone oil.
(13) The gel composition of any of (1) to (12) above, further characterized by containing a higher fatty acid (C).
(14) A method for manufacturing any one of the gel compositions of (1) to (12) above comprising the steps of:
   1) uniformly hot melting the gelling agent, oily base, and any other additives; and
   2) stirring and cooling.
(15) An aromatic cosmetic composition characterized by comprising the gel composition of any of (1) to (13) above.
(16) A cosmetic characterized by comprising the gel composition of any of (1) to (13) above.
(17) An aromatic characterized by comprising the gel composition of any of (1) to (13) above.

### Best Mode of Implementing the Invention

Modes of implementing the present invention are described in detail below.

In the present invention, at least one N-acyl amino acid (A) denoted by general formula (1) below is employed as a gelling agent component of the gel composition: 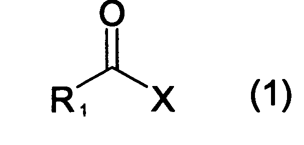 (wherein R₁ denotes a straight or branched-chain saturated hydrocarbon group having from 1 to 25 carbon atoms or an unsaturated hydrocarbon group optionally containing an aromatic ring and X denotes an aliphatic or aromatic α-, β-, or γ-amino acid).

Examples of the amino acid moiety (X in general formula (1)) of N-acyl amino acid (A) are glutamic acid, aspartic acid, and other acidic amino acids; glycine, alanine, valine, leucine, isoleucine, serine, threonine, cysteine, methionine, asparagine, glutamine, methylglycine, β-alanine, N-methyl- β-alanine, γ-aminobutyric acid, taurine, N-methyltaurine, phenylalanine, tyrosine, proline, tryptophan, and other N-acyl neutral amino acids; and lysine, arginine, ornithine, and other basic amino acids. These amino acids may be employed singly or in combinations of two or more. Of these amino acids, the acyl neutral amino acids are preferred, with alanine being of even greater preference. The optical activity of the amino acid employed is not specifically limited; a racemic, L-form, or D-form may be employed. However, from the perspective of obtaining a gel that is highly stable at elevated temperatures, optical activity (L-form or D-form) is desirable.

As regards the acyl group of the N-acyl amino acid, R₁ in general formula (1) denotes an acyl group in the form of a straight or branched-chain saturated hydrocarbon group with 1 to 25 carbon atoms or an unsaturated hydrocarbon group optionally containing an aromatic ring, with R₁ preferably having 5 to 21, more preferably, 7 to 17, carbon atoms. An "unsaturated hydrocarbon group optionally containing an aromatic ring" means that some or all of the unsaturated bonds may be derived from aromatic rings. Preferred acyl groups are aliphatic acyl groups such as 2-ethylhexanoyl, octanoyl, decanoyl, lauroyl, myristoyl, palmitoyl, and stearoyl. Of these, a stearoyl group is particularly preferred.

N-acyl amino acid (A) of the present invention can be manufactured by N-acylating an amino acid by the usual methods. For example, it can be obtained by the Schotten-Baumann reaction in which a fatty acid halide is reacted with an amino acid in the presence of a basic catalyst such as sodium hydroxide.

Oily base (B) employed in the gel composition of the present invention is not specifically limited other than that it thoroughly solubilize or uniformly disperse the above gelling agent when heated and form a gel when cooled to room temperature.

Specific examples are silicone oils selected from among methylpolysiloxanes, high-polymer methylsiloxanes, polyoxyethylene-methylpolysiloxane copolymers, polyoxypropylene-methylpolysiloxane copolymers, poly(oxyethylene, oxypropylene)-methylpolysiloxane copolymers, other ether-denatured silicones, stearoxymethylpolysiloxane, stearoxytrimethylsilane, methylhydrogenpolysiloxane, octamethylpolysiloxane, decamethylpolysiloxane, decamethylcyclopentasiloxane, octamethylcyclotetrasiloxane, tetrahydrotetramethylcyclotetrasiloxane, methylcyclopolysiloxane, cyclopentasiloxane, dodecamethylcyclohexasiloxane, other cyclic siloxanes, 1,1,5,5-tetraphenyltrisiloxane, methylphenylpolysiloxane, trimethylsiloxysilicic acid, aminoethylaminopropylsiloxane-dimethylsiloxane copolymer, other amino-denatured silicones, silanol-denatured polysiloxanes, alkoxy-denatured polysiloxanes, fatty acid-denatured polysiloxanes, fluorine-denatured polysiloxanes, epoxy-denatured polysiloxanes, alkoxy-denatured polysiloxane perfluoropolyethers, polyvinyl acetate dimethylpolysiloxanes, and mixtures thereof; higher alcohols such as cetyl alcohol, isostearyl alcohol, lauryl alcohol, hexadecyl alcohol, and octadecyl alcohol; esters such as myristyl myristate, hexyl laurate, decyl oleate, isopropyl myristate, hexyldecyl dimethyloctanoate, glycerin monostearate, diethyl phthalate, ethylene glycol monostearate, octyl oxystearate; hydrocarbons such as liquid paraffin, vaseline, and squalane; waxes such as lanolin, reduced lanolin, and carnauba wax; oils such as mink oil, cacao oil, coconut oil, palm kernel oil, tsubaki oil, sesame oil, castor oil, and olive oil; and ethylene-α-olefin co-oligomers.

The blending ratio of component (A) employed in the present invention is not specifically limited. However, it desirably constitutes 0.01 to 30 weight percent, preferably 0.1 to 20 weight percent, and more preferably, 0.5 to 10 weight percent of the total quantity of components (A) and (B). When the blending ratio of N-acyl amino acid is less than 0.01 weight percent, adequate gel strength is precluded, and in some cases, a portion of the oily base exudes from the gel. When 30 weight percent is exceeded, dissolution or uniform dispersion in the oily base becomes difficult, and in some cases, solids precipitate in the gel composition.

A total quantity of components (A) and (B) of from 1 to 99.99 weight percent of the total composition (the total of components (A) and (B) and other components) is commonly employed in the present invention. When the blending ratio of the oily base is less than 1 weight percent or exceeds 99 percent, the obtaining of an adequately strong gel is precluded, the N-acyl amino acid does not completely dissolve, and when the gel composition is employed as a cosmetic or the like, good skin feel tends to be substantially lost.

As an example of a preferred mode of the present invention consisting of the two components of gelling agent (A) and oily base (B), gelling agent (A) (that is, N-acyl amino acid (A)) is in the form of N-acyl-L-alanine and oily base (B) is in the form of a silicone oil or a hydrocarbon oil. In the mode of greatest preference, gelling agent (A) is N-acyl-L-alanine and oily base (B) is silicone oil, particularly a silicone oil selected from among methylpolysiloxane, high polymer methylpolysiloxane, octamethyltrisiloxane, de[ca]methyltetrasiloxane, decamethylcyclopentasiloxane, octamethylcyclotetrasiloxane, methylcyclopolysiloxane, dodecamethylcyclohexasiloxane, and methylphenylpolysiloxane.

A higher fatty acid (C) is employed in the gel composition of the present invention with the goals of partially inhibiting crystallization of the N-acyl amino acid in the gel composition and achieving a creamy, smooth sensation when the gel composition is applied to the skin or hair. There are no specific limitations beyond achieving these goals. Specific examples are: 2-ethylhexanoic acid, capric acid, caprylic acid, lauric acid, myristic acid, palmitic acid, stearic acid, coconut oil fatty acid, beef tallow fatty acid, behenic acid, oleic acid, and isostearic acid. These may be employed singly or in combinations of two or more. From the perspective of achieving a good feeling of spreadability and the like, lauric acid, myristic acid, palmitic acid, stearic acid, coconut oil fatty acid, behenic acid, oleic acid, and isostearic acid are preferred, with lauric acid, myristic acid, palmitic acid, stearic acid, and isostearic acid being of particular preference.

The blending ratio of component (C) of the present invention is 0.01 to 30 weight percent of component (A). At less than 0.01 weight percent, there is an inadequate preventive effect on the precipitation of N-acyl amino acid (A), and an adequately creamy, smooth feeling cannot be obtained. At greater than 30 weight percent, the obtaining of a gel of adequate strength tends to be impossible. From the perspective of stabilizing and maintaining creamy, smooth characteristics, 0.1 to 20 weight percent is preferred, and I to 10 weight percent is of even greater preference.

The method of manufacturing the gel composition of the present invention is not specifically limited. For example, when manufacturing a gel composition comprised of the three components of gelling agent (A), oily base (B), and higher fatty acid (C), the targeted gel composition can be obtained by heating the three components to about 50°C or above with stirring until gelling agent (A) dissolves or melts, and then cooling the mixture with stirring or by letting it stand. In this process, when applying heat to melt gelling agent (A) in the form of N-acyl-L-alanine or the like, oily base (B), and higher fatty acid (C) and then cooling them while stirring to manufacture a gel composition, it is desirable for there not to be partial crystallization of gelling agent (A) in the form of N-acyl-L-alanine or the like so as to achieve a gel composition that has good spreadability and is attractive in appearance. A gel composition comprised of the two components of gelling agent (A) and oily base (B) can be manufactured by omitting higher fatty acid (C) in the above-described process of manufacturing a gel composition comprised of three components.

The gel composition of the present invention can be made into a variety of cosmetic and aromatic products as is, or by further blending in various components employed in cosmetics and aromatics. Accordingly, the above-described two-component and three-component gel compositions of the present invention not only (narrowly) cover two-component gel compositions comprising components (A) and (B) and three component gel compositions additionally comprising component (C), but also (broadly) cover gel compositions obtained by adding various other components to these narrowly defined gel compositions.

When employing the gel composition of the present invention as a composition for aromatic cosmetics, various chelating agents and the like are examples of the various components that can be blended in to inhibit discoloration of and odor generation by the composition. The chelating agent is not specifically limited; preferred chelating agents are triethylenetetramine, 1,1,1-trifluoro-3,2'-thenoylacetone, thioglucolic acid, tartaric acid, succinic acid, 8-quinolinol, pyridine-2,6-dicarboxylic acid, pyridine, 1,10-tenanthroline, lactic acid, 8-hydroxyquinoline-5-sulfonic acid, glycine, 2,2'-pyridylethylenediamine, aurintricarboxylic acid, xylenol orange, 5-sulfosalicylic acid, salicylic acid, pyrocatechol-3,5-disulfonate, 4,5-dihydroxybenzene-1,3-disulfonic acid, 1,2-diaminocyclohexane-N,N,N',N'-tetraacetic acid, citric acid, oxalate, nitrilotriacetic acid, ethylenediamine-N,N,N',N'-tetraacetic acid, acetylacetone, salts thereof, and mixtures of the same.

Oily base (B) gelling agents other than above-described N-acyl amino acid (A) may be combined as additional blending components. Examples are N-acyl-L-glutamic acid dialkylamide, polyamide resin, 12-hydroxystearic acid, sodium stearate, dibenzylidene-D-sorbitol, and fatty acid dextrin.

As a further blending component, water may be blended in to obtain compositions for aromatic cosmetics in the form of W/O or O/W emulsions.

To the extent that the effect of the present invention is not compromised, further blending components in the form of surfactants, various additives, various powders, and the like can be suitably blended into the composition for aromatic cosmetics of the present invention.

Examples of surfactants are anionic surfactants such as N-long chain acyl acidic amino acid salts, N-long chain acyl neutral amino acid salts, other N-long chain acyl amino acid salts, N-long chain fatty acid acyl-N-methyltaurine salts, alkyl sulfates and alkylene oxide adducts thereof, fatty acid amide ether sulfates, metal salts and weak base salts of fatty acids, sulfosuccinic acid surfactants, alkylphosphate and alkyleneoxide adducts thereof, and alkyl ether carboxylic acids; nonionic surfactants such as ether surfactants in the form of glycerin ethers and alkylene oxide adducts thereof, ester surfactants in the form of glycerin esters and alkylene oxide adducts thereof, ether ester surfactants such as sorbitan esters and alkylene oxide adducts thereof, polyoxyalkylene fatty acid esters, glycerin esters, fatty acid polyglycerin esters, sorbitan esters, sucrose fatty acid esters, and other ester surfactants, alkyl glucosides, hardened castor oil pyroglutamic acid diester and ethylene oxide adducts thereof, fatty acid alkanol amides, and other nitrogenous nonionic surfactants; cationic surfactants such as alkyl ammonium chloride, dialkyl ammonium chloride, other aliphatic amine salts, quaternary ammonium salts thereof, benzalkonium salts, other aromatic quaternary ammonium salts, and fatty acid acyl arginine esters; and amphoteric surfactants such as carboxybetaine, other betaine surfactants, aminocarboxylic acid surfactants, and imidazoline surfactants.

Examples of various additives are glycine, L-alanine, DL-alanine, serine, threonine, arginine, glutamic acid, aspartic acid, leucine, valine, and other amino acids; glycerin, ethylene glycol, 1,3-butylene glycol, propylene glycol, isoprene glycol, and other polyhydric alcohols; polyglutamic acid, polyaspartic acid, other polyamino acids, salts thereof, polyethylene glycol, various forms of gum arabic, alginic acid, xanthan gum, hyaluric acid, salts of hyaluric acid, chitin, chitosan, water-soluble chitin, carboxyvinyl polymers, carboxymethyl cellulose, hydroxyethyl cellulose, hydroxypropyl trimethyl ammonium chloride, polydimethylmethylenepiperidium chloride, polyvinyl pyrrolidone derivative quaternary ammonium, cationized proteins, collagen decomposition products and derivatives thereof, acylated proteins, polyglycerin, and other water-soluble macromolecules; mannitol, other sugar alcohols, and alkylene oxide adducts thereof; ethanol, propanol, and other lower alcohols; plant and animal extracts, nucleic acids, vitamins, enzymes, anti-inflammatory agents, bactericides, preservatives, antioxidants, ultraviolet absorbants, chelating agents, antiperspirants, pigments, oxide dyes, organic and inorganic powders, pH adjusting agents, pearling agents, and moisturizers.

Examples of various powders are nylon beads, silicone beads, vegetable powders, nylon powders, metallic fatty acid soaps, yellow iron oxide, red iron oxide, black iron oxide, chromium oxide, cobalt oxide, carbon black, ultramarine blue, iron blue, zinc oxide, titanium oxide, zirconium oxide, silicon oxide, aluminum oxide, cerium oxide, titanium mica, boron nitride, barium sulfate, calcium carbonate, magnesium carbonate, aluminum silicate, magnesium silicate, silicon carbide, pigments, lakes, sericite, mica, talc, kaolin, plate barium silicate, butterfly-shaped barium silicate, microparticulate titanium oxide, microparticulate zinc oxide, microparticulate iron oxide, acyl lysine, acyl glutamate, acyl arginine, acyl glycine, and other acyl amino acids. Surface processing with silicone, fluorine compounds, silane coupling agents, silane processed organic titanate, acylated lysine, fatty acids, metallic soaps, oils, amino acids, and the like can also be conducted.

It is not particularly difficult to manufacture a gel composition (broadly defined) comprising the two components of component (A) and (B), or comprising three components in the form of these two components with the addition of component (C), into which the above-described various components have been blended. For example, this can be done as described above by combining all of the components, heating the mixture while stirring until gelling agent (A) dissolves or melts, and then cooling the mixture with stirring or by letting it stand.

The gel composition of the present invention may be employed as one of the starting materials of aromatic cosmetic compositions such as cosmetics and aromatics. The form of the aromatic cosmetic is not specifically limited. However, in the case of cosmetics, the preferred forms are powders, pastes, and solids. Specific examples are foundations, facial powder, rouge, eye shadow, other cosmetic products; body cosmetics such as body powders; foundation cosmetics such as eaux de toilette, lotions, beauty treatments, skin creams, suncare products; make-up removers, face-cleansing foams, and other cleaning agents; rinses, treatments, styling agents, antiperspirants, bath treatments, hair dyes, and other hair cosmetics; and solid, cream, and liquid aromatic cosmetics. Aromatics are desirably in the form of creams or gels. Specific examples are aromatics for automobile and home use.

Further, the advantageous gel characteristics of the gel composition of the present invention may be exploited for use in utility paints, art paints, solid utility paints, solid art paints, oil-based inks, and other stationery products and utility paints; general merchandise and toiletries such as pocket heaters, ice packs, paint cleaners, and car waxes; modifiers such as polyolefin resins; poultices, agricultural chemicals in gel form, solid insecticides, and other medical drugs and agricultural chemicals; rust preventing oils, greases, lubricating oils, cutting oils, and other mechanical oils; asphalt modifiers; and photographic emulsions.

### [Embodiments]

The present invention will be described in greater detail below through embodiments; however, the present invention is not limited thereto.

### <Manufacturing Example 1: Manufacturing of N-lauroyl-L-alanine>

To 110 g of L-alanine were added 290 g of water and 230 g of t-BuOH, after which an aqueous solution comprising 27 percent of sodium hydroxide was added to dissolve the mixture. The pH was adjusted to 11. The solution was cooled to 10°C, and 269 g of lauroyl chloride and an aqueous solution comprising 27 percent of sodium hydroxide were simultaneously added dropwise while maintaining the pH at 11. With the conclusion of the reaction, the acylated reaction solution was neutralized with 75 percent sulfuric acid, adjusted to pH 2, and subjected to oil separation at 60°C to remove the aqueous layer. To the oil layer obtained were added 757 g of water. The mixture was stirred, after which oil separation was conducted. A suitable quantity of isopropyl alcohol (IPA) was added to the oil layer and the mixture was cooled, yielding 320 g of a white, crystalline substance. This substance was recrystallized from IPA solvent and the white crystalline substance obtained was dried at 50°C under reduced pressure, yielding N-lauroyl-L-alanine.

### <Manufacturing Example 2>

To 33.23 g of L-alanine were added 185.88 g of water, after which 54.1 g of an aqueous solution comprising 27 percent sodium hydroxide was added to dissolve the mixture. The pH was adjusted to 11. The solution was heated to 35°C and 81.6 g of lauroyl chloride and an aqueous solution comprising 27 percent of sodium hydroxide were simultaneously added dropwise while maintaining the pH at 11. With the conclusion of the reaction, the acylated reaction solution was neutralized with 75 percent sulfuric acid, adjusted to pH 2, and subjected to oil separation at 70°C to remove the aqueous layer. To the oil layer obtained were added 203 g of water. The mixture was stirred, after which oil separation was conducted. The oil layer was dried under reduced pressure (50°C, reduced pressure), yielding 94.32 g of a white, crystalline substance (N-lauroyl-L-alanine).

### <Embodiment 1>

A prescribed quantity of N-acyl amino acid was weighed out and charged to a 50 mL vial. An oil solution was then added. The mixture was heated to 100°C while stirring. Once the N-acyl amino acid had completely dissolved, the mixture was cooled to room temperature. The form of the gel composition (including skin feel) obtained was confirmed. The results are given in Table 1.

**[Table 1]**

| N-acyl amino acid | Lauroyl-L-alanine | | | |
|---|---|---|---|---|
| Blending ratio (wt%) | 1 | 3 | 5 | 10 |
| Oil solution | Δ | ○ | ○ | ○ |
| Isopropyl myristate | Δ | ○ | ○ | ○ |
| Liquid paraffin*1 | Δ | ○ | ○ | ○ |
| Cyclomethicone*2 | Δ | ○ | ○ | ○ |
| Dimethicone*3 | ○ | ○ | ○ | ○ |
| X: Did not gel | | | | |
| Δ: Partially gelled or solution increased in viscosity | | | | |
| ○: Gelled (into cream or solid) | | | | |

| | | | | |
|---|---|---|---|---|
| *1: Smoil P55 made by Matsumura Yushi | | | | |
| *2: SH245C made by Tore - Dow Corning Silicone K.K. | | | | |
| *3: SH200C made by Tore - Dow Corning Silicone K.K. | | | | |

### <Embodiment 2>

A prescribed quantity of N-acyl amino acid was weighed out (to achieve a 5 percent concentration of 5-acyl amino acid in the targeted gel composition) and charged to a 30 mL vial. An oil solution was then added. The mixture was heated to 90 to 100°C with stirring. Once the N-acyl amino acid had completely dissolved, the form of the gel composition (including skin feel) obtained was confirmed. The results are given in Table 2.

**[Table 2]**

| Oil solution | N-acyl amino acid | | | | |
|---|---|---|---|---|---|
| | Lauroyl-L-alanine | Lauroyl-L-methionine | Lauroyl-L-phenylalanine | Lauroyl-DL-alanine | Palmitoyl-L-valine |
| Dodecane | - | ○ | ○ | ○ | - |
| Dimethicone*1 | ○ | - | - | - | Δ |
| -: Not implemented | | | | | |
| X: Did not gel | | | | | |
| Δ: Partially gelled or solution increased in viscosity | | | | | |
| ○: Gelled (into cream or solid) | | | | | |

| | | | | | |
|---|---|---|---|---|---|
| *1: SH200C made by Tore - Dow Corning Silicone K.K. Only N-lauroyl-L-alanine was stable when stored for one week at 50°C. | | | | | |

### <Embodiment 3>

A prescribed quantity of N-acyl amino acid was weighed out (to achieve a 3 percent concentration of 5-acryl amino acid in the targeted gel composition) and charged to a 30 mL vial. An oil solution was then added. The mixture was heated to 90 to 100°C with stirring. Once the N-acyl amino acid had completely dissolved, the form of the gel composition (including skin feel) obtained was confirmed. The results are given in Table 3.

**[Table 2]**

| Oil solution | Lauroyl-L-alanine | |
|---|---|---|
| | Cooled with vigorous stirring | Cooled without stirring |
| Liquid paraffin*1 | ○ | Δ |
| Dichloromethicone*2 | ○ | Δ |
| Dimethicone*3 | ○ | Δ |
| X: Did not gel | | |
| Δ: Partially gelled or solution increased in viscosity | | |
| ○: Gelled (into cream or solid) | | |

| | | |
|---|---|---|
| *1: Smoil P55 made by Matsumura Yushi | | |
| *2: SH245C made by Tore - Dow Corning Silicone K.K. | | |
| *3: SH200C made by Tore - Dow Corning Silicone K.K. | | |

### <Embodiment 4>

Prescribed quantities of N-acyl amino acid and fatty acid were weighed out and charged to a 50 mL vial. An oil solution was then added. The mixture was heated to 90 to 100°C. Once the N-acyl amino acid had completely dissolved, the mixture was cooled to room temperature, with or without vigorous stirring. The appearance and softness when applied to the skin of the gel compositions obtained were evaluated by an expert panel. The evaluations were conducted based on the following criteria. An average of 2.6 or greater was denoted by **ⓞ**, 2.2 to 2.6 by ○, 1.8 to 2.2 by ○, and 1.8 or less by X. The results are given in Table 4.

### Evaluation criteria

### [Appearance]

- 3:: Uniform and smoothly creamy
- 2:: Nearly uniformly creamy, but some precipitation of solid matter observed.
- 1:: Precipitation of solid matter observed

### [Spreadability when applied to skin]

- 3:: Extremely smooth
- 2:: Generally smooth, but with some roughness
- 1:: Felt quite rough

**[Table 4]**

| Blending ratio (wt%) | Embodiment 4-1 | Embodiment 4-2 |
|---|---|---|
| Lauroyl-L-alanine | 5 | 4.65 |
| Lauric acid | - | 0.35 |
| Dimethicone* 1 | 95 | 95 |
| Evaluation results | | |
| Appearance | ○ | **ⓞ** |
| Spreadability when applied to skin | ○ | **ⓞ** |

| | | |
|---|---|---|
| *1: SH200C made by Tore - Dow Corning Silicone K.K. | | |

As the result of evaluation by an expert five-person panel, the cream gel obtained in Embodiment 4-2 was found to be creamier in appearance and to have better smoothness when applied to the skin than the cream gel obtained in Embodiment 4-1.

### <Embodiment 5: A comparison test with conventional gelling agents>

Gel 1 based on the N-lauroyl-L-alanine (5 weight percent) obtained in Manufacturing Example 1 and liquid paraffin (95 weight percent, Smoil P55 made by Matsumura Yushi) was compared with gel 2 based on a conventional gelling agent in the form of lauroyl-glutamic acid dibutyl amide (GP-1)(1.5 weight percent) and liquid paraffin (98.5 weight percent). While gel 1 was a paste exhibiting good spreadability, and was thus suited to creams, gel 2 was a waxlike, overall solid, and was thus suited to lipstick. Marked differences were thus exhibited.

### <Embodiment 6: Manufacturing of aromatic cosmetic gel compositions>

A 0.25 g quantity of the N-lauroyl-L-alanine obtained in Manufacturing Example 1 and 4.75 g of liquid paraffin ("Morecos P55" made by Matsumura Yushi) were charged to a vial. The mixture was heated to 90°C with stirring to melt the components. The mixture was then left standing to cool to room temperature, and complete gelling was confirmed.

The gel composition obtained was translucent, did not produce a clammy or clogging sensation when applied to the skin, had good spreadability, and was found to be well-suited to gel compositions for cosmetics.

### <Embodiment 7: Manufacturing of gel compositions for aromatic cosmetics>

A 0.2325 g quantity of the N-lauroyl-L-alanine obtained in Manufacturing Example 1, 0.0175 g of lauric acid, and 4.75 g of dimethicon ("SH200C" made by Tore - Dow Corning Silicon K.K.) were charged to a 50 mL vial, heated to 90°C with stirring, and dissolved. The solution was then vigorously stirred by hand while cooling to room temperature. Overall gelling was confirmed.

The gel composition obtained was a white-to-translucent cream, did not produce a clammy or clogging sensation when applied to the skin, had good spreadability, and was found to be well-suited to gel compositions for cosmetics.

| <Formulation Example 1: Handcream> | |
|---|---|
| Liquid paraffin (* 1) | 30 weight percent |
| Dimethicon (*2) | 65 weight percent |
| Lauroyl-L-alanine | 5 weight percent |

| | |
|---|---|
| (*1: "Morecos P55" made by Matsumura Yushi) | |
| (*2: "SH200C" made by Tore - Dow Corning Silicone K.K.) | |

When a handcream was prepared by the usual methods from the above formulation, it exhibited the properties of a white-to-translucent creaminess without a clammy or clogging sensation and with good spreadability.

| <Formulation Example 2: Handcream> | |
|---|---|
| Liquid paraffin | 30 weight percent |
| Dimethicon | 65 weight percent |
| Lauroyl-L-alanine | 4.65 weight percent |
| Lauric acid | 0.35 weight percent |
| (*1: "Morecos P55" made by Matsumura Yushi) | |
| (*2: "SH200C" made by Tore - Dow Corning Silicone K.K.) | |

When a handcream was prepared from the above formulation, it exhibited the properties of a white-to-translucent, rather delicate creaminess, without a clammy or clogging sensation, that was smooth to the touch.

### Industrial Applicability

Based on the present invention, it is possible to obtain a gel composition in the form of a translucent to milky white cream to solid with an attractive appearance. When employed in aromatic cosmetic compositions such as cosmetics, this gel composition yields an aromatic cosmetic product that does not produce a clammy or clogging feeling and that affords good spreadability.

## Claims

1. A gel composition **characterized by** comprising at least one N-acyl amino acid (A) denoted by general formula (1) below and at least one oily base (B): 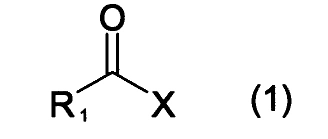 (wherein R₁ denotes a straight or branched-chain saturated hydrocarbon group having from 1 to 25 carbon atoms or an unsaturated hydrocarbon group optionally containing an aromatic ring and X denotes an aliphatic or aromatic α-, β-, or γ-amino acid).

2. The gel composition of claim 1 above further **characterized in that** an optically active form (L-form or D-form) of N-acyl amino acid (A) is employed.

3. The gel composition of claim 1 or 2 above, further **characterized in that** the X in general formula (1) of N-acyl amino acid (A) denotes a neutral amino acid.

4. The gel composition of any of claims 1 to 3 above, further **characterized in that** N-acyl amino acid (A) is N-acyl-L-alanine.

5. The gel composition of any of claims I to 4 above, further **characterized in that** in N-acyl amino acid (A) of general formula (1), R₁ denotes a straight or branched-chain saturated hydrocarbon group with 5 to 21 carbon atoms, or an unsaturated hydrocarbon group optionally comprising an aromatic ring.

6. The gel composition of any of claims 1 to 5 above, further **characterized in that** in N-acyl amino acid (A) of general formula (1), R₁ denotes a straight or branched-chain aliphatic hydrocarbon group with 7 to 17 carbon atoms.

7. The gel composition of any of claims 1 to 6 above, further **characterized in that** N-acyl amino acid (A) is selected from among N-2-ethylhexanoyl-L-alanine, N-capryloyl-L-alanine, N-caproyl-L-alanine, N-lauroyl-L-alanine, N-myristoyl-L-alanine, and N-palmitoyl-L-alanine.

8. The gel composition of any of claims 1 to 7 above, further **characterized in that** N-acyl amino acid (A) is an N-lauroyl amino acid.

9. The gel composition of any of claims 1 to 8 above, further **characterized in that** N-acyl amino acid (A) is N-lauroyl-L-alanine.

10. The gel composition of any of claims 1 to 9 above, further **characterized in that** oily base (B) is liquid at room temperature.

11. The gel composition of any of claims 1 to 10 above, further **characterized in that** oily base (B) is a hydrocarbon oil or silicone oil.

12. The gel composition of any of claims 1 to 11 above, further **characterized in that** oily base (B) is a silicone oil.

13. The gel composition of any of claims 1 to 12 above, further **characterized by** containing a higher fatty acid (C).

14. A method for manufacturing any one of the gel compositions of claims 1 to 13 above comprising the steps of:
1) uniformly hot melting the gelling agent, oily base, and any other additives; and
2) stirring and cooling.

15. An aromatic cosmetic composition **characterized by** comprising the gel composition of any of claims 1 to 13 above.

16. A cosmetic **characterized by** comprising the gel composition of any of claims I to 13 above.

17. An aromatic **characterized by** comprising the gel composition of any of claims 1 to 13 above.
